# EUROPEAN PATENT APPLICATION

(11) **EP 2 778 171 A1**
(43) Date of publication of application: **17.09.2014**
(21) Application number: 13159392.3
(22) Date of filing: 15.03.2013
(51) Int. Cl.: C07H 15/252

(54) **Crystalline monohydrate of epirubicin hydrochloride**

(71) Applicant: Synbias Pharma Ltd., 83085 Donetsk (UA)
(72) Inventor: Zabudkin, Alexander, 83055 Donetsk (UA); Matvyeyev, Alexey, 83114 Donetsk (UA); Matvienko, Victor, 83114 Donetsk (UA)
(74) Representative: Danner, Stefan

(57) **Abstract**

The present invention relates to a crystalline monohydrate of epirubicin hydrochloride having water content in the range between 2.7% and 3.5% (w/w) and being devoid of residual solvents as well as to a corresponding method for its production. The method comprises: (a) adding at least a first solvent and at least a second solvent to epirubicin hydrochloride, wherein the first solvent is a linear or branched C₄ to C₅ alcohol, and the second solvent is a linear or branched C₂ to C₃ alcohol; (b) adjusting in the solution obtained the water content to an amount in the range between 7% and 12% (w/w); and (c) heating the mixture to a temperature of 70°C to 90°C in order to allow crystallization.

## Description

### FIELD OF THE INVENTION

The present invention relates to a crystalline monohydrate of epirubicin hydrochloride and a corresponding method for its production. More particularly, the invention is directed to a crystalline monohydrate of epirubicin hydrochloride having a water content of 2.7% to 3.5% (w/w) and being devoid of residual solvents.

### BACKGROUND OF THE INVENTION

Anthracyclines represent a class of naturally occurring bioactive compounds derived from the bacterial genus *Streptomyces.* Several anthracyclines, including daunorubicin, idarubicin, doxorubicin, epirubicin, and carminomycin, were clinically demonstrated to be effective antineoplastic agents that can be employed for the treatment of a wide range of cancers including breast cancer, lung cancer, and hematological malignancies such as leukemias and lymphomas. In addition, members of this class of compounds were also shown to be useful in bone marrow transplants and during stem cell transplantation.

Epirubicin is the 4-epimer of doxorubicin and a semi-synthetic derivative of daunorubicin and represented by the following formula:

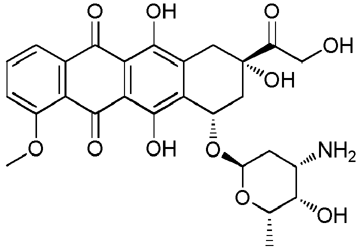

Although the precise mechanisms of its cytotoxic properties have not yet been completely elucidated, it is believed that epirubicin forms complexes with DNA by intercalation of its planar rings between nucleotide base pairs, with consequent inhibition of nucleic acid (DNA and RNA) and protein synthesis, by triggering DNA cleavage via topoisomerase II. Epirubicin and its addition salts, such as epirubicin hydrochloride, are used as a component of adjuvant treatment of breast cancer.

Various methods for the production of epirubicin hydrochloride are currently known in the art. However, amorphous epirubicin hydrochloride (e.g., prepared as described in U.S. Patent 4,861,870) is generally considered as not suitable for pharmaceutical applications due to its significant hygroscopy and chemical instability during storage, with accumulation of the aglycone doxorubicinone.

Certain crystalline polymorphs of epirubicin hydrochloride, being characterized by particular X-ray diffraction patterns, have previously become available and are described *inter alia* in the patent documents: WO 2005/004805 A1; WO 2010/039159 A1; WO 2011/118929 A2; and WO 2012/163508 A1. Although the various X-ray diffraction patterns show a high degree of similarity, the corresponding methods of production are considerably diverse. Notably, the synthesis schemes described in the two first-mentioned documents have subsequently been indicated not to reliably result in a crystalline product (cf. WO 2012/163508 A1; paragraphs [0007] and [0069]).

The crystalline polymorphs of epirubicin hydrochloride described in WO 2005/004805 A1 and WO 2010/039159 A1 (designated "type II") as well as WO 2011/118929 A2 (designated "form A" to "form D") have been shown to exhibit an improved thermal stability during storage of up to six months.

However, several of the production methods employed in the above patent documents involve the use of significant amounts of solvents. It is well established in the art that anthrycyclines form solvates (cf., e.g., Courseille, C. et al. (1979) Acta Cryst. B35, 764-767). Such solvates are known to show an improved thermal stability as compared to the respective "pure" compound. The above-referenced production schemes do not address the problem of the formation of solvates. Hence, it is not fully apparent whether the improved thermal stability observed for the above-referenced polymorphs of epirubicin hydrochloride can in fact be assigned to the (pure) compound *per se* or is influenced by the formation of solvates (i.e. the presence of residual solvents in the final product).

Beyond, it is also evident that solvates are more difficult to be controlled for its hygroscopic properties as solvates have an increased tendency to absorb water. The presence of residual solvents (at least in readily detectable amounts) should also have influence on product safety (cf. *inter alia* ICH [International Conference on Harmonization of Technical Requirements for Registration of Pharmaceuticals for Human Use] Guideline Q3C (R5)). For example, the production of crystal forms A, C, and D according to WO 2011/118929 A2 involves the use of class 2 solvents (as defined by ICH), such as acetonitrile and N,N-dimethyl-formamide, whose use in pharmaceutical products should be limited due to their inherent toxicity.

Thus, there is still a need for a crystalline form of epirubicin hydrochloride that overcomes the above limitations, in particular with regard to long-term product stability and product safety.

In particular, there remains a need for a compound having an improved thermal stability and a prolonged shelf-life, while also exhibiting a reduced hydroscopic capacity (i.e. stability against water) and being virtually devoid of residual solvents.

Accordingly, it is an object of the present invention to provide such crystalline form of epirubicin hydrochloride as well as a corresponding method for its production.

### SUMMARY OF THE INVENTION

In one aspect, the present invention relates to a method for producing crystalline epirubicin hydrochloride, comprising: (a) adding at least a first solvent and at least a second solvent to epirubicin hydrochloride, wherein the first solvent is a linear or branched C₄ to C₅ alcohol, and the second solvent is a linear or branched C₂ to C₃ alcohol; (b) adjusting in the solution obtained in step (a) the water content to an amount in the range between 7% and 12% (w/w); and (c) heating the solution obtained in step (b) to a temperature of 70°C to 90°C in order to allow crystallization; wherein the crystalline epirubicin hydrochloride produced is a monohydrate having a water content in the range between 2.7% and 3.5% (w/w) and being devoid of residual solvents.

In preferred embodiments, the water content in step (b) of the method is adjusted to an amount in the range between 8% and 11% (w/w).

In further preferred embodiments, the first solvent is selected from the group consisting of 1-butanol, 2-butanol, isobutanol, tert-butanol, and mixtures thereof; and the second solvent is selected from the group consisting of 1-propanol, isopropanol, ethanol, and mixtures thereof. Particularly preferably, the first solvent is 1-butanol and the second solvent is 1-propanol.

In particular embodiments, the volume ratio of the first solvent to the second solvent is in the range between 1:1 and 2:1 (v/v).

In further particular embodiments, the final concentration of epirubicin hydrochloride in the solution of step (b) is in the range between 10 g/l and 100 g/l.

In preferred embodiments, the solution in step (c) is heated to a temperature of 75°C to 85°C.

In specific embodiments, the method further comprises seeding of the solution obtained in step (b) in order to induce crystallization.

In other specific embodiments, the method further comprises purifying the crystals obtained in step (c).

In preferred embodiments, the water content of the crystalline monohydrate of epirubicin hydrochloride produced is in the range between 2.8% and 3.3% (w/w).

In another aspect, the present invention relates to a crystalline monohydrate of epirubicin hydrochloride being produced by the method as described herein above.

In a further aspect, the present invention relates to a crystalline monohydrate of epirubicin hydrochloride having a water content in the range between 2.7% and 3.5% (w/w), and being devoid of residual solvents. Preferably, the crystalline monohydrate of epirubicin hydrochloride has a water content in the range between 2.8% and 3.3% (w/w).

In particular embodiments, the crystalline monohydrate of epirubicin hydrochloride is further characterized by a powder X-ray diffraction pattern comprising peaks at average diffraction angles (2Θ) of 5.1 °, 9.1 °, 13.6°, 22.1 °, 22.5°, and 24.0° (each ± 0.1°).

A further aspect of the present invention relates to the crystalline monohydrate of epirubicin hydrochloride as described herein above for use in the prevention and treatment of cancer.

### BRIEF DESCRIPTION OF THE DRAWINGS

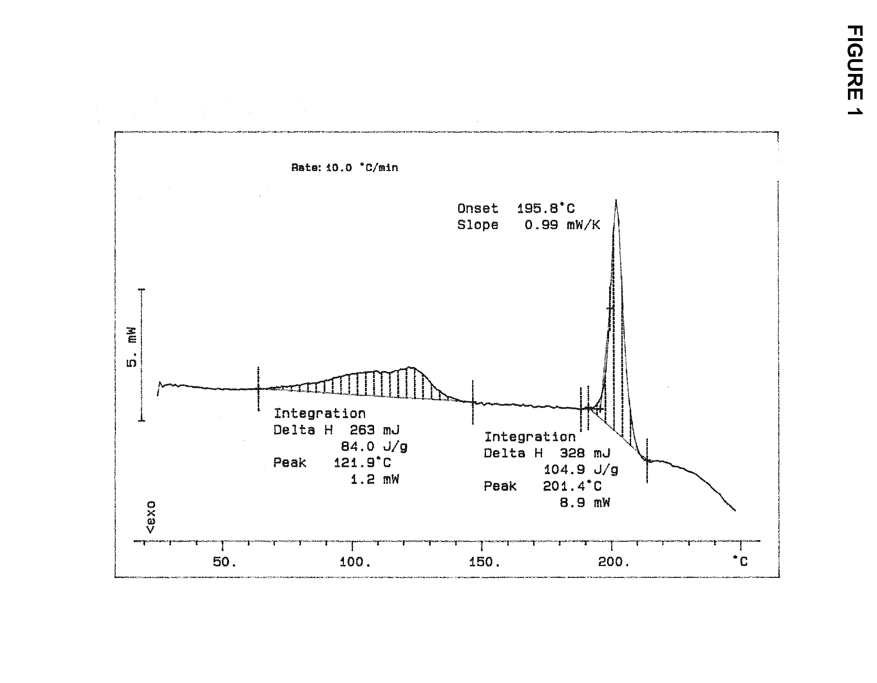

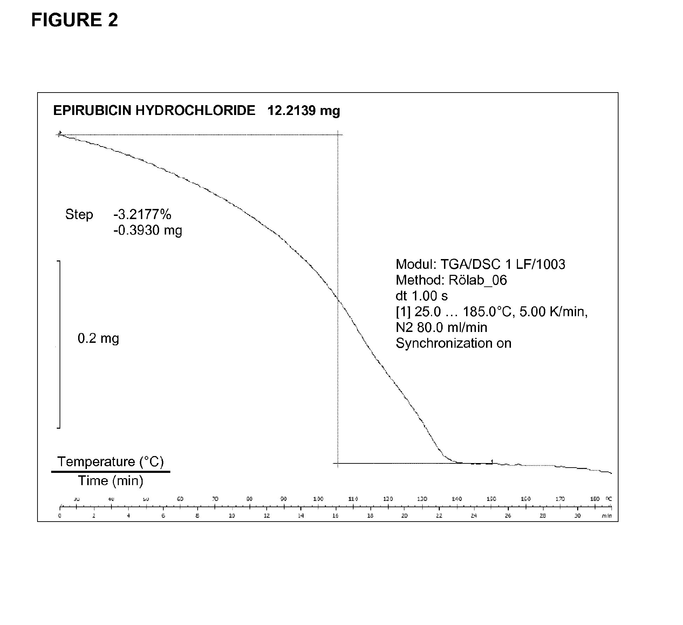

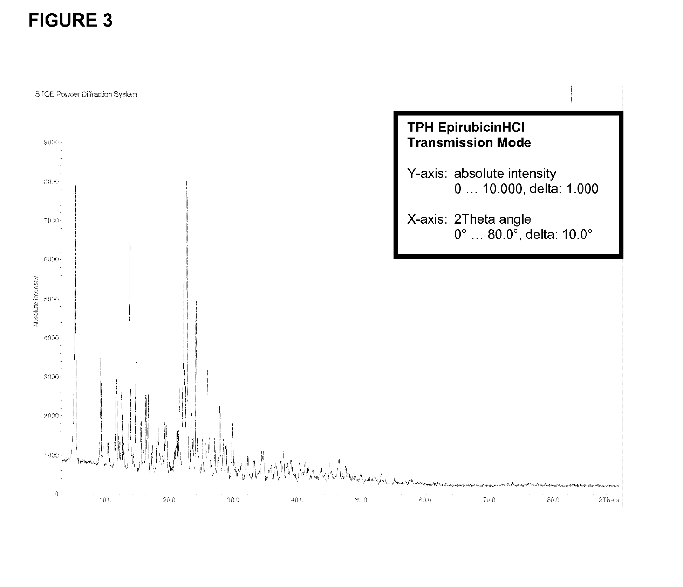

**FIGURES 1-3** illustrate the functional characterization of an exemplary crystalline monohydrate of epirubicin hydrochloride of the present invention, being prepared according to Example 1. **FIG. 1** shows the results of a representative DSC analysis for determining the melting point; **FIG. 2** the results of a representative thermogravimetric analysis for determining the water content; and **FIG. 3** the results of a representative X-ray powder diffraction analysis.

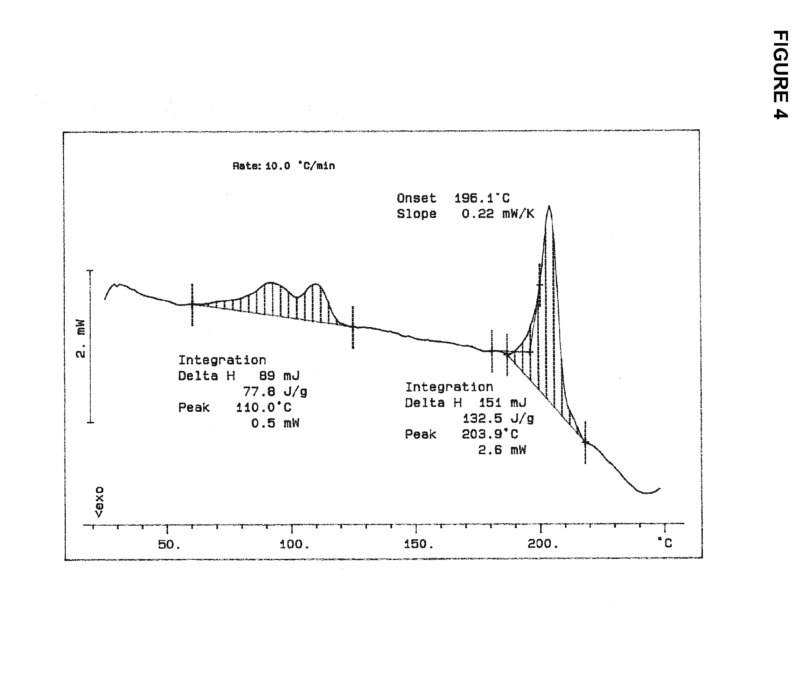

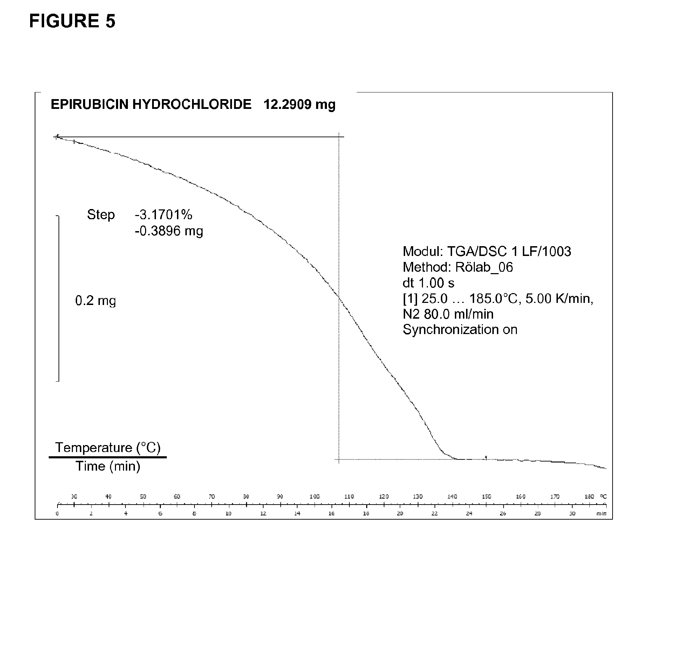

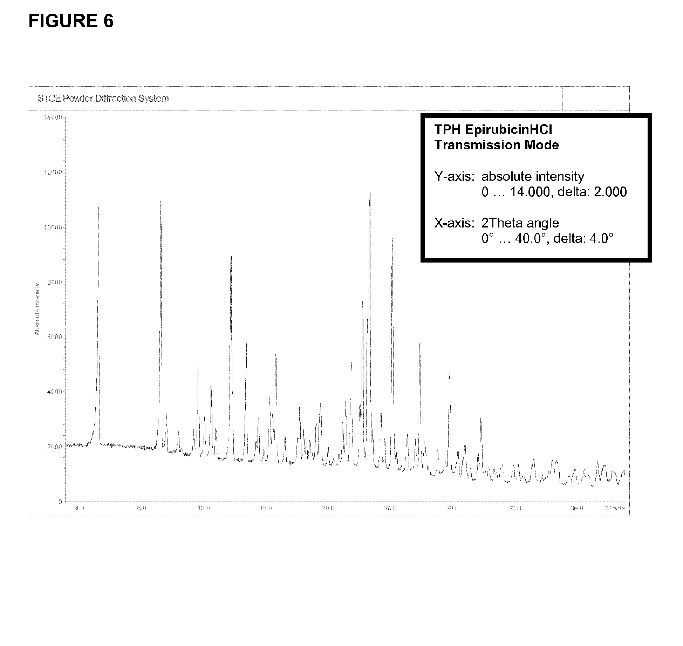

**FIGURES 4-6** illustrate the functional characterization of an exemplary crystalline monohydrate of epirubicin hydrochloride of the present invention, being prepared according to Example 2. **FIG. 4** shows the results of a representative DSC analysis for determining the melting point; **FIG. 5** the results of a representative thermogravimetric analysis for determining the water content; and **FIG. 6** the results of a representative n X-ray powder diffraction analysis.

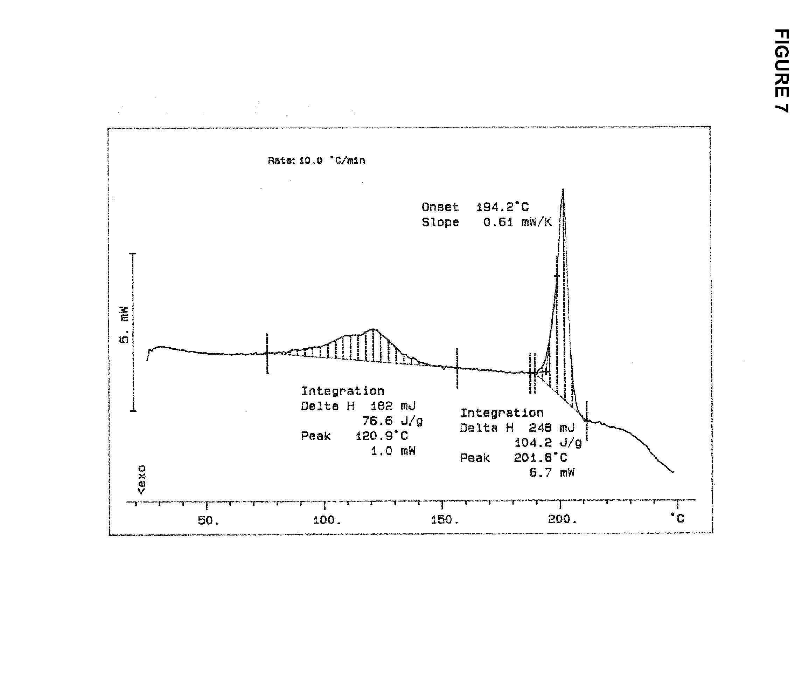

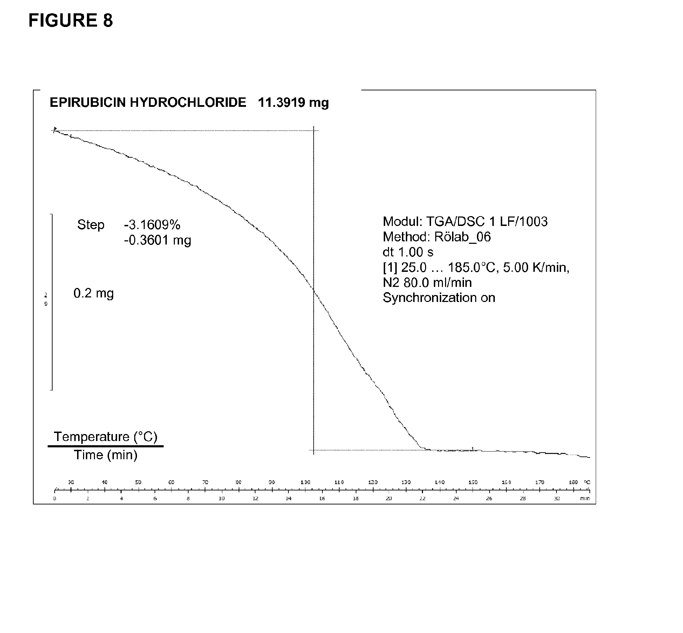

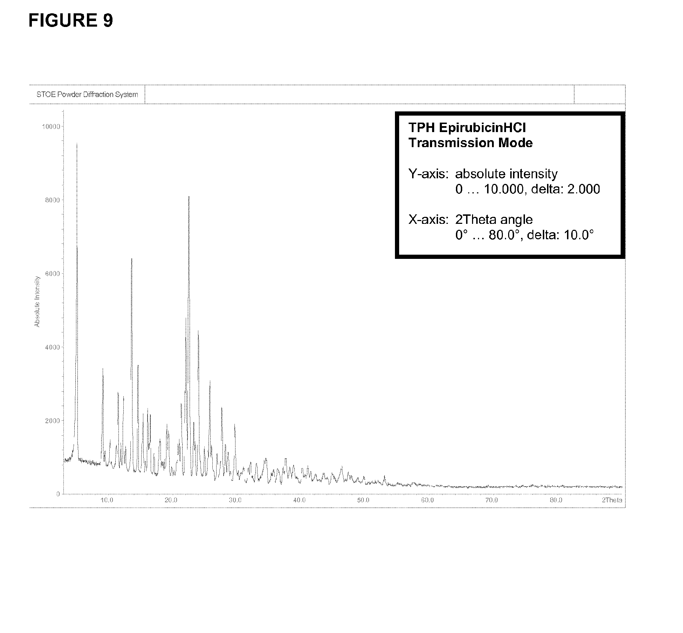

**FIGURES 7-9** illustrate the functional characterization of an exemplary crystalline monohydrate of epirubicin hydrochloride of the present invention, being prepared according to Example 3. **FIG. 7** shows the results of a representative DSC analysis for determining the melting point; **FIG. 8** the results of a representative thermogravimetric analysis for determining the water content; and **FIG. 9** the results of a representative X-ray powder diffraction analysis.

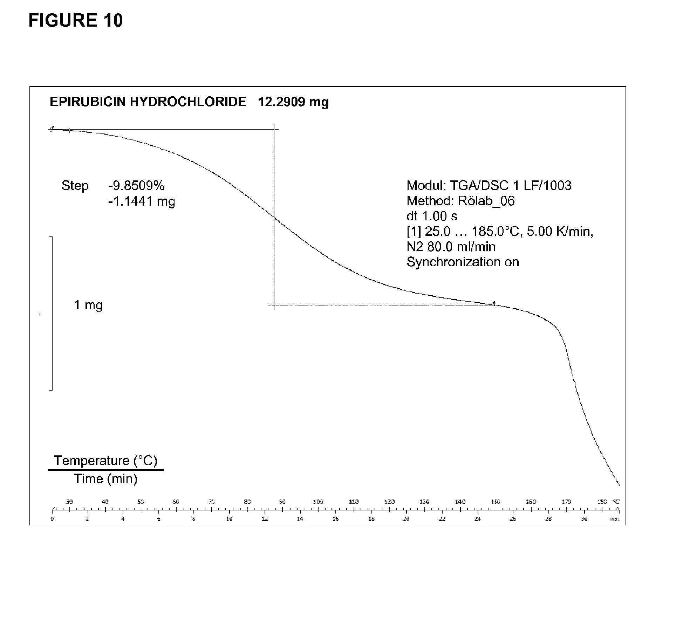

**FIGURE 10** illustrates a representative thermogravimetric analysis for determining the water content of a crystalline trihydrate of epirubicin hydrochloride, being prepared according to Example 3.

### DETAILED DESCRIPTION OF THE INVENTION

The present invention is related to the unexpected finding that the provision of epirubicin hydrochloride in form of a monohydrate having a water content in the range between 2.7% and 3.5% (w/w) combines an improved thermal stability and a long-term shelf-life with advantageous hygroscopic properties as well as an superior product safety (due to the absence of residual solvents). The adjustment of the water content of the solvent mixture prior to crystallization to an amount in the range between 7% and 12% (w/w) in combination with a comparably high crystallization temperature has been found to be a characteristic technical feature in obtaining the crystalline monohydrate.

Without intending to be bound by a particular theory the presence of a certain amount of residual water in the crystal polymorph is considered to interfere with the incorporation of solvent molecules in the crystal structure and/or to displace them from the crystal structure without having any significant adverse effect on the stability of the crystals against heat or water, with the latter effect being particularly surprising.

Notably, when preparing epirubicin hydrochloride in form of a trihydrate by increasing the amount of water present in the solvent mixture, the superior properties identified for the monohydrate form were no longer observed. Particularly, the trihydrate form is considerably less stable against heat and water than the monohydrate form. Accordingly, the superior properties are specifically associated with the monohydrate form of epirubicin hydrochloride.

The present invention will be described in the following with respect to particular embodiments and with reference to certain drawings but the invention is to be understood as not limited thereto but only by the appended claims. The drawings described are only schematic and are to be considered non-limiting.

Where the term "comprising" is used in the present description and claims, it does not exclude other elements or steps. For the purposes of the present invention, the term "consisting of" is considered to be a preferred embodiment of the term "comprising". If hereinafter a group is defined to comprise at least a certain number of embodiments, this is also to be understood to disclose a group, which preferably consists only of these embodiments.

Where an indefinite or definite article is used when referring to a singular noun e.g. "a", "an" or "the", this includes a plural of that noun unless specifically stated otherwise.

In case, numerical values are indicated in the context of the present invention the skilled person will understand that the technical effect of the feature in question is ensured within an interval of accuracy, which typically encompasses a deviation of the numerical value given of ± 10%, and preferably of ± 5%.

Furthermore, the terms first, second, third, (a), (b), (c), and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

Further definitions of term will be given in the following in the context of which the terms are used. The following terms or definitions are provided solely to aid in the understanding of the invention. These definitions should not be construed to have a scope less than understood by a person of ordinary skill in the art.

In one aspect, the present invention relates to a method for producing crystalline epirubicin hydrochloride, comprising:
(a) adding at least a first solvent and at least a second solvent to epirubicin hydrochloride, wherein the first solvent is a linear or branched C₄ to C₅ alcohol, and the second solvent is a linear or branched C₂ to C₃ alcohol;
(b) adjusting in the solution obtained in step (a) the water content to an amount in the range between 7% and 12% (w/w); and
(c) heating the solution obtained in step (b) to a temperature of 70°C to 90°C in order to allow crystallization;
wherein the crystalline epirubicin hydrochloride produced is a monohydrate having a water content in the range between 2.7% and 3.5% (w/w) and being devoid of residual solvents.

For producing the crystalline monohydrate of epirubicin hydrochloride the compound is mixed with at least a first solvent and at least a second solvent. Epirubicin hydrochloride may be provided as a solid, in amorphous or in any crystalline form. Alternatively, epirubicin hydrochloride may be provided in form of a solution or a suspension, such as an aqueous solution. The compound can be produced by chemical synthesis or by fermentation or is commercially available from various suppliers.

Typically, epirubicin hydrochloride is provided as a solid and then dissolved in water, for example, in a concentration in the range between 10 g/l to 100 g/l. The pH value of this solution may be in the range between 3.0 and 5.0, preferably between 3.5 and 4.5. In particular embodiments, the water is then removed until a gel state of the solution is achieved. For example, removal of water can be accomplished by evaporation (e.g. using a rotor-evaporator) under low pressure and at a temperature of 30°C to 45°C, preferably at a temperature of 35°C to 40°C.

The "at least first solvent", as defined herein, is a linear or branched C₄ to C₅ alcohol, that is, an alcohol having four or five carbon atoms, *inter alia* including the C₄ alcohols 1-butanol, 2-butanol, isobutanol, and tert-butanol as well as the C₅ alcohols 1-pentanol, 2-pentanol, 3-pentanol, 2-methyl-1-butanol, 2-methyl-2-butanol, 3-methyl-1-butanol, 3-methyl-2-butanol, and 2,2-dimethyl-1-propanol. The term "at least a first solvent", as used herein, also refers to mixtures of one or more C₄ alcohols, such as (1-butanol and isobutanol) or (1-butanol, isobutanol, and tert-butanol), mixtures of one or more C₅ alcohols, such as (1-pentanol and 2-pentanol) or (1-pentanol, 2-pentanol, and 2-methyl-1-butanol), as well as mixtures of one or more C₄ and C₅ alcohols, such as (1-butanol and 1-pentanol) or (1-butanol, 2-butanol, and 1-pentanol).

The "at least a second solvent", as defined herein, is a linear or branched C₂ to C₃ alcohol, that is, an alcohol having two or three carbon atoms, *inter alia* including the C₂ alcohol ethanol and the C₃ alcohols 1-propanol and isopropanol. The term "at least a second solvent", as used herein, also refers to mixtures of one or more C₂ and/or C₃ alcohols, such as (ethanol and 1-propanol) or (1-propanol and isopropanol) or (ethanol, 1-propanol, and isopropanol)

In preferred embodiments of the method, the first solvent is selected from the group consisting of 1-butanol, 2-butanol, isobutanol, tert-butanol, and mixtures thereof; and wherein the second solvent is selected from the group consisting of 1-propanol, isopropanol, ethanol, and mixtures thereof. For example, the at least first solvent may be 1-butanol and the second solvent ethanol. Or, the at least first solvent may be tert-butanol and the at least second solvent a mixture of 1-propanol and ethanol. In particularly preferred embodiments, the first solvent is 1-butanol and the second solvent is 1-propanol.

In specific embodiments, epirubicin hydrochloride may be mixed with at least a third or at least a forth solvent, wherein the at least third or at least forth solvent are selected from linear or branched C₁ to C₁₀ alcohols, and particularly from linear or branched C₁ to C₆ alcohols.

The at least first solvent and the at least second solvent may be added to the epirubicin hydrochloride simultaneously or sequentially in any order. Typically, the at least first solvent and the at least second solvent are added sequentially, starting with the at least first solvent.

In particular embodiments, after addition of the at least first solvent, the water being present in the mixture is removed (as complete as possible) until a solid precipitate is formed. Removal of water can be accomplished by evaporation (e.g. using a rotor-evaporator) under low pressure and at a temperature of 30°C to 45°C, preferably at a temperature of 35°C to 40°C.

After addition of the at least second solvent (either simultaneously or sequentially) the resulting mixture is typically incubated, optionally at an elevated temperature, until complete dissolution is accomplished, that is, any solid precipitates being present have been dissolved. To this end, the mixture may be heated (preferably under stirring) to a temperature of 40°C to 55°C, preferably to a temperature of 45°C to 50°C.

In further particular embodiments, the volume ratio of the first solvent to the second solvent is in the range between 1:1 and 2:1 (v/v). In exemplary embodiments, this volume ratio is 1:1, 1.2:1, 1.4:1, 1.6:1, 1.8:1 or 2:1 (v/v). However, other volume ratios of the first solvent to the second solvent are possible as well, such as a higher excess than 2:1 (v/v) of the first solvent (e.g., 2.5:1, 3:1 or 5:1 (v/v)) or an excess of the second solvent (e.g., 1:1.2, 1:2 or 1:3 (v/v)).

Subsequently, the water content in the solution obtained after addition of the at least first solvent and the at least second solvent is adjusted to an amount in the range between 7% and 12% (w/w). This adjustment can be accomplished by the addition of missing water or the removal of excess water, for example by evaporation (cf. above). The water content can be determined by various methods established in the art, for example by Karl-Fischer titration (Fischer, K. (1935) Angew. Chem. 48, 394-396), wherein a coulometric or volumetric titration may be performed, or by thermogravimetric analysis (reviewed, e.g., in Coats, A.W. and Redfern, J.P. (1963) Analyst 88, 906-924).

In exemplary embodiments, the water content of the solution is in the range between 7.2% and 12% (w/w), between 7.5% and 12% (w/w), between 8% and 11.8% (w/w), between 7% and 11.5% (w/w) or between 7.5% and 11% (w/w), for example 7.5%, 8.0%, 8.5%, 9.0%, 9.5%, 10.0%, 10.5%, 11.0%, 11.5% or 12% (w/w).

In preferred embodiments of the method, the water content in the solution is adjusted to an amount in the range between 8% and 11% (w/w) or between 8% and 10% (w/w) or between 9% and 11% (w/w) or between 8% and 12% (w/w), for example 8.0%, 8.2%, 8.4%, 8.6%, 8.8%, 9.0%, 9.2%, 9.4%, 9.6%, 9.8%, 10.0%, 10.2%, 10.4%, 10.6%, 10.8% or 11.0% (w/w).

In particular embodiments, the final concentration of epirubicin hydrochloride in the solution obtained after the adjustment of the water content is in the range between 10 g/l and 100 g/l, for example 10 g/l, 20 g/l, 50 g/l, 80 g/l or 100 g/l. However, it is also possible to use solutions having a higher concentration of epirubicin hydrochloride, for example up to 120 g/l, 150 g/l or 200 g/l.

The pH value of the solution obtained after the adjustment of the water content may be in the range between 2.5 and 6.0 or between 3.0 and 5.5. In preferred embodiments, the pH value is adjusted to a value in the range between 3.5 and 4.5, for example to 4.0.

The solution obtained (i.e. after addition of the at least first solvent and the at least second solvent to epirubicin hydrochloride and complete dissolution) is then heated to a temperature of 70°C to 90°C in order to allow crystallization. Preferably, the solution is heated to a temperature of 75°C to 85°C, or particularly preferably to a temperature of 78°C to 85°C or of 78°C to 82°C, for example 75°C, 76°C, 77°C, 78°C, 79°C, 80°C, 81°C, 82°C, 83°C, 84°C or 85°C. The mixture is preferably heated under stirring.

The incubation at a temperature of 70°C to 90°C is continued until a solid precipitate (i.e. crystals of epirubicin hydrochloride) has been formed, for example, for up to 1 hour, for up to 2 hours, for up to 4 hours or for up to 6 hours. Stirring may be continued during incubation.

In specific embodiments, the method further comprises seeding of the solution obtained after the adjustment of the water content in order to induce crystallization. That is, one or more small epirubicin crystals are added to the solution, from which larger crystals are grown.

In other specific embodiments, the method further comprises purifying the crystals obtained during crystallization. Typically, such purification involves the separation of the crystals from the other components of the mixture, which is usually accomplished by filtration or distillation. Furthermore, the purification process may involve a washing step with a suitable washing solution such as an alcohol or a ketone.

In particular embodiments of the methods, the optional purification process comprises a fluid extraction process using a supercritical fluid, that is, a substance at a temperature and pressure above its critical point, where distinct liquid and gas phases do not exist. Supercritical fluids to be used herein include *inter alia* nitrous oxide, propane, ethylene, fluorocarbons, and carbon dioxide, with the latter one being preferred. The fluid extraction process may be performed at a temperature between 25-55°C (e.g., between 40-45°C) and a pressure between 10-60 MPa (e.g., between 40-55 MPa) for a period of 2-4 hours (e.g., 3 hours) at a flow rate in the range between 200-2000 ml/min (e.g., between 500-1000 ml/min).

Finally, the crystals may be dried according to established protocols, optionally under vacuum. For example, the crystals obtained can be air-dried at a temperature of 30°C to 40°C.

Functional characterization of the resulting crystals revealed that they represent a monohydrated form of epirubicin hydrochloride (i.e. one water molecule per molecule of epirubicin hydrochloride) having a water content in the range between 2.7% and 3.5% (w/w). The water content can be determined as described above, for example by Karl-Fischer titration or by thermogravimetric analysis.

In preferred embodiments of the method, the water content of the crystalline monohydrate of epirubicin hydrochloride is in the range between 2.8% and 3.3% (w/w), for example 2.8%, 2.9%, 3.0%, 3.1%, 3.2% or 3.3% (w/w).

Furthermore, the functional characterization of the crystalline monohydrate of epirubicin hydrochloride produced demonstrated that the compound is devoid of residual solvents. The term "devoid of", as used herein, refers to an amount of residual solvents in the crystals being less than 0.5% (w/w), preferably less than 0.3% (w/w) and particularly preferably less than 0.25% (w/w). The amount of residual solvents present in the crystals can be determined by using various well established methods, such as gas chromatography.

In another aspect, the present invention relates to a crystalline monohydrate of epirubicin hydrochloride being produced by the method as described herein above.

In yet another aspect, the present invention relates to a crystalline monohydrate of epirubicin hydrochloride having a water content in the range between 2.7% and 3.5% (w/w), and being devoid of residual solvents, that is, comprises an amount of residual solvents being less than 0.5% (w/w) (cf. above). Preferably, the water content of the crystalline monohydrate is in the range between 2.8% and 3.3% (w/w) (cf. above as well as the exemplary thermogravimetric analyses in **FIGURES 2****,** **5****,** and **8**).

In particular embodiments, the crystalline monohydrate of epirubicin hydrochloride is further characterized by a particular powder X-ray diffraction pattern, as exemplified by the analyses shown in **FIGURES 3****,** **6**, and **9**, wherein the diffraction pattern comprises characteristic peaks at average diffraction angles (2Θ) of 5.1 °, 9.1°, 13.6°, 22.1 °, 22.5°, and 24.0° (each ± 0.1°).

In specific embodiments, the crystalline monohydrate of epirubicin hydrochloride of the present invention is further characterized by a particular differential scanning calorimetry (DSC) diagram, as exemplified by the analyses shown in **FIGURES 1****,** **4**, and **7**, comprising a maximum intensity in the temperature range of 195°C to 205°C with an average peak (i.e. melting point) between 201 °C and 204°C.

Finally, a further aspect of the present invention relates to the crystalline monohydrate of epirubicin hydrochloride as described herein above for use in the prevention and treatment of cancer. Particularly, the crystalline monohydrate of epirubicin hydrochloride is used for the treatment of breast cancer.

The invention is further described by the figures and the following examples, which are solely for the purpose of illustrating specific embodiments of this invention, and are not to be construed as limiting the scope of the invention in any way.

### EXAMPLES

### MATERIALS AND METHODS

Differential scanning calorimetry (DSC; for determining the melting point) was performed by precise sample weighing into alumina crucibles (70 µl, hermetically closed with an alumina lid) by using a calibrated ultra-micro balance. Measurement: Mettler TC11-TA-Processor with DSC 30 module or Mettler DSC 25 with silver-oven and ceramic sensor crystal and Mettler TC15A-TA-Controller (25°C to 250°C, 10°C/min). Purging gas: N₂, 80 ml/min, mass-flow controlled Calibration: Performed directly before the sample measurement with ultrapure Indium (In) as a reference material (temperature scale, heat-flow scale).

Coulometric Karl-Fischer titration (KFT; for determining the water content) was performed with an external drying oven (Metrohm 707 KF; operated at 140°C with N₂ purge). The samples were precisely weighed on an analytical balance (10⁻⁵ g).

Thermogrvimetric analysis (TG analysis; for determining the water content) was performed by precise sample weighing into alumina crucibles (100 µl, sealed with an alumina lid having a laser drilled 50 µm hole) by using a calibrated ultra-micro balance. Measurement: Mettler TGA/DSC1, large oven; gas control-box (purging gas: N₂, 80 ml/min, mass-flow controlled).

HPLC and GC analyses were performed according to standard protocols.

X-ray powder diffraction analysis was performed using a STOE-STADI P transmission diffractometer with the following setup: nCu-Kα₁ radiation (λ = 1.54056 A), U = 40 kV, I = 35 mA, primary beam monochromator (curved Ge(111)), linear position sensitive detector, slits: 1 mm, d = 8 mm, angle region: 2θ = 3 to 90°, step width Δ2θ = 0.02°, 25 s/0.2° step. The powder is originally filled between two mylar foils and then into the sample holder having a d = 8 mm mask.

### EXAMPLE 1

(1) 500.0 g of amorphous epirubicin hydrochloride (91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) Absolute ethanol in the amount of 5 I was added to the 1-butanol solution. The mixture was heated until the solid precipitate was re-dissolved. The water content of the 1-butanol/ethanol solution was adjusted by means of evaporation to 8% to 10% (w/w) (as determined by Karl-Fisher titration). Subsequently, evaporation was stopped and the solution was stirred at a temperature of 75°C until a solid precipitate was formed.
(4) Crystals of epirubicin hydrochloride were collected by filtration, washed in 500 ml of acetone and air-dried at a temperature of 30°C to 35°C. 423 g of crystalline epirubicin hydrochloride were obtained (overall yield: 92.9%).

Functional characterization of the compound obtained:

| | |
|---|---|
| Melting point (DSC analysis): 201.4°C (cf. **FIG. 1**) | |
| Water content: | 3.11% (w/w) (KFT) |
| | 3.22% (w/w) (TG analysis; cf. **FIG. 2**) |
| | Calculated value for monohydrate: 3.01% (w/w)) |
| Epirubicin HCI content (HPLC analysis): 96.4% (w/w) | |
| Content of residual solvents (GC analysis): 0.26% (w/w) | |
| X-ray powder diffraction analysis (cf. **FIG. 3**): characteristic peaks at diffraction angles (2Θ) of 5.1 °, 9.1°, 13.6°, 22.1 °, 22.5°, and 24.0° | |

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

### EXAMPLE 2

(1) 500.0 g of amorphous epirubicin hydrochloride (content: 91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) 1-propanol in the amount of 5 I was added to the 1-butanol solution. The mixture was heated until the solid precipitate was re-dissolved. The water content of the n-butanol/1-propanol solution was adjusted by means of evaporation to 9% to 10% (w/w) (as determined by Karl-Fisher titration). Subsequently, evaporation was stopped and the solution was stirred at a temperature of 78°C until a solid precipitate was formed.
(4) Crystals of epirubicin hydrochloride were collected by filtration, washed in 500 ml of acetone and air-dried at a temperature of 30°C to 35°C. 435 g of crystalline epirubicin hydrochloride were obtained (overall yield: 95.6%).

Functional characterization of the compound obtained:

| | |
|---|---|
| Melting point (DSC analysis): 203.9°C (cf. **FIG. 4**) | |
| Water content: | 3.11% (w/w) (KFT) |
| | 3.17% (w/w) (TG analysis; cf. **FIG. 5**) |
| | Calculated value for monohydrate: 3.01% (w/w)) |
| Epirubicin HCI content (HPLC analysis): 96.1% (w/w) | |
| Solvent content (GC analysis): 0.23% (w/w) | |
| X-ray powder diffraction analysis (cf. **FIG. 6**): characteristic peaks at diffraction angles (2Θ) of 5.1 °, 9.1°, 13.6°, 22.1 °, 22.5°, and 24.0° | |

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

### EXAMPLE 3

(1) 500.0 g of amorphous epirubicin hydrochloride (content: 91.0% (w/w), as determined by HPLC analysis), were dissolved in 10 I of water. The water was evaporated on a rotor-evaporator under low pressure at a temperature of 40°C until a gel state of the solution was achieved.
(2) 1-butanol in an amount of 10 I was added to the solution and evaporation was continued until a solid precipitate was formed.
(3) A mixture of ethanol and 1-propanol (1:1 (v/v)) in the amount of 5 I was added to the 1-butanol solution. The mixture was heated until the solid precipitate was re-dissolved. The water content of the resulting solution was adjusted by means of evaporation to 8% to 10% (w/w) (as determined by Karl-Fisher titration). Subsequently, evaporation was stopped and the solution was stirred at a temperature of 75°C until a solid precipitate was formed.
(4) Crystals of epirubicin hydrochloride were collected by filtration, washed in 500 ml of acetone and air-dried at a temperature of 30°C to 35°C. 431 g of crystalline epirubicin hydrochloride were obtained (overall yield: 94.7%).

Functional characterization of the compound obtained:

| | |
|---|---|
| Melting point (DSC analysis): 201.6°C (cf. **FIG. 7**) | |
| Water content: | 3.11% (w/w) (KFT) |
| | 3.16% (w/w) (TG analysis; cf. **FIG. 8**) |
| | Calculated value for monohydrate: 3.01% (w/w)) |
| Epirubicin HCI content (HPLC analysis): 97.0 % (w/w) | |
| Solvent content (GC analysis): 0.21% (w/w) | |
| X-ray powder diffraction analysis (cf. **FIG. 9**): characteristic peaks at diffraction angles (2Θ) of 5.1 °, 9.1°, 13.6°, 22.1 °, 22.5°, and 24.0° | |

The experimental data reveal that the compound obtained is epirubicin hydrochloride (HPLC) having a specifically defined crystalline structure (X-ray), a water content being indicative of a monohydrate form (KFT and TG), and being devoid of residual solvents (GC).

The functional data obtained in Examples 1-3 show a very high degree of consistency being indicative of the production of the same compound.

### EXAMPLE 4 (COMPARATIVE EXAMPLE)

(1) 500.0 g of crystalline epirubicin hydrochloride were dissolved in 5 I of water. The water was evaporated on a rotor-evaporator under low pressure at a temperature of 40°C until a gel state of the solution was achieved.
(2) The gel solution of epirubicin hydrochloride was poured into 9 I of acetone.
(3) Precipitated crystals of epirubicin hydrochloride were collected by filtration, washed in 500 ml of acetone and air-dried at a temperature of 30°C to 35°C. 512 g of precipitated crystals were obtained. HPLC analysis confirmed the presence of epirubicin hydrochloride.
(4) Determination of the water content (calculated value for the trihydrate: 9.03% (w/w)

| | |
|---|---|
| Analysis: | 9.81% (w/w) (KFT) |
| | 9.85% (w/w) (TG analysis; cf. **FIG. 10**) |

The experimental data reveal that the crystalline epirubicin hydrochloride obtained has a water content being indicative of a trihydrate form.

### EXAMPLE 5

The crystalline monohydrate of epirubicin hydrochloride prepared according to Example 1 was exposed to a temperature of 25°C, 40°C, and 100°C, respectively, for the time period (in months) indicated. The testing was performed as described in the ICH guidelines. The amount of the degradation product (i.e. doxorubicinone) produced as well as that of intact epirubicin hydrochloride (both in % (w/w)) was determined by HPCL analysis ("LT" = less than). The results are summarized in the following table 5-1.

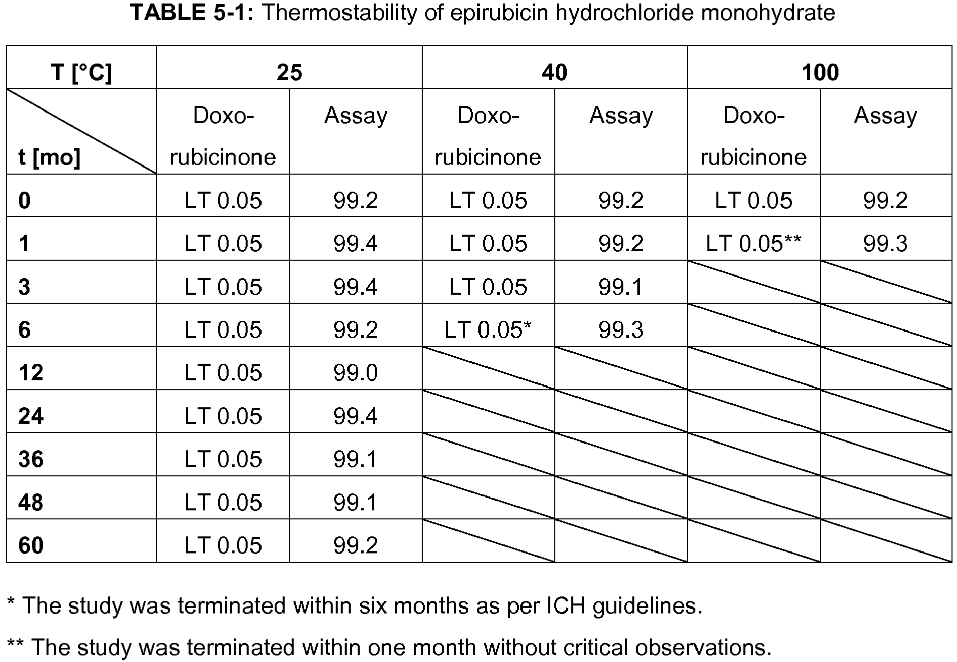

The results demonstrate an exceptional high stability of the crystalline monohydrate produced, not only with regard to the overall shelf-life (stability over a time period of (at least) 5 years at a temperature of 25°C) but also with regard to thermostability (stability over a time period of (at least) 6 months at a temperature of 40°C).

Additional analyses using the crystalline monohydrate of epirubicin hydrochloride prepared according to Examples 2 and 3, respectively, revealed almost identical results (not shown).

The same analysis was performed with the crystalline trihydrate of epirubicin hydrochloride prepared according to Example 4. The results are summarized in the following table 5-1.

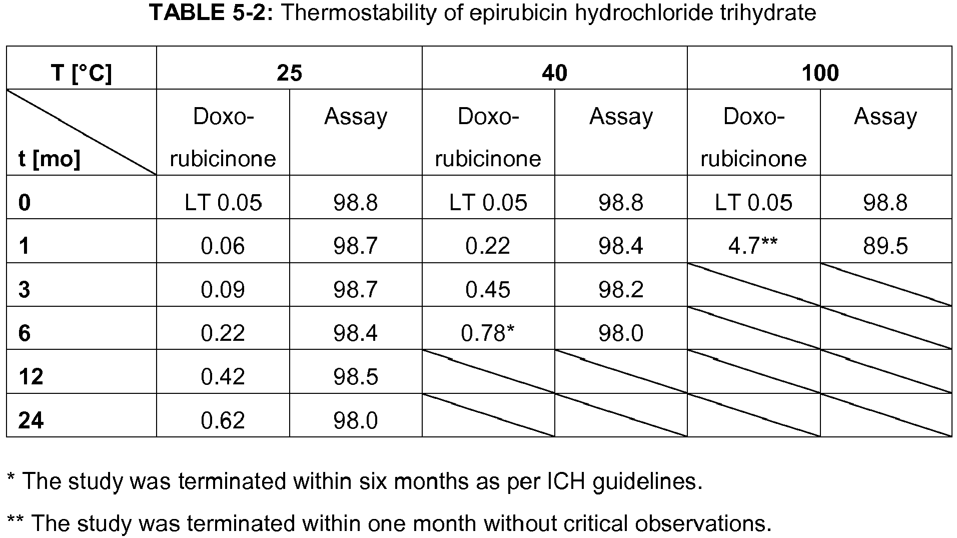

The data show significant degradation of the trihydrate form even within six months storage at a temperature of 25°C. Accumulation of the doxorubicinone aglycone drastically increases with elevated temperature. Hence, the superior properties of the crystalline epirubicin hydrochloride of the present invention are an inherent feature of the monohydrate form obtained.

### EXAMPLE 6

The crystalline monohydrate of epirubicin hydrochloride prepared according to Example 1 was exposed to a temperature of 25°C or 40°C and a relative humidity of 40-90%, respectively, for the time period (in days) indicated. The testing was performed as described in the ICH guidelines. The respective water contents of the samples (in % (w/w)) were measured by means of Karl-Fischer titration and the results summarized in the following table 6-1.

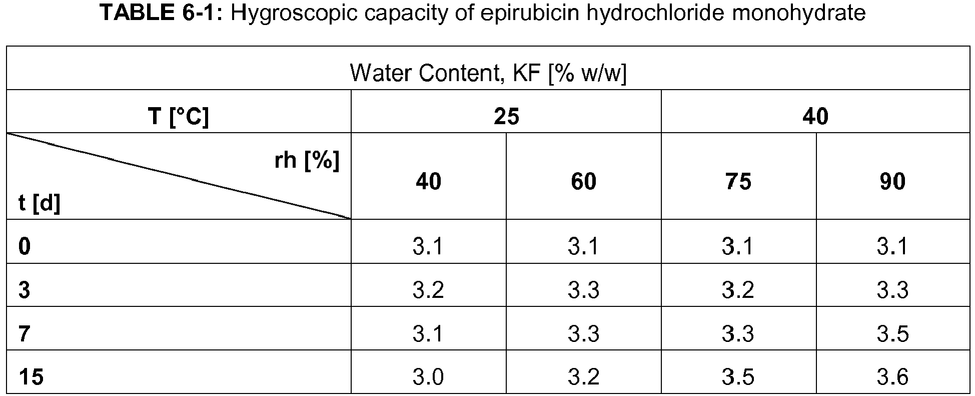

The experimental data show that the crystalline monohydrate of epirubicin hydrochloride produced does not exhibit any significant hygroscopic capacity. Even when exposed to 90% relative humidity and 40°C for 15 days the water absorption was surprisingly small.

Additional analyses using the crystalline monohydrate of epirubicin hydrochloride prepared according to Examples 2 and 3, respectively, revealed almost identical results (not shown).

The same analysis was performed with the crystalline trihydrate of epirubicin hydrochloride prepared according to Example 4. The results are summarized in the following table 6-2.

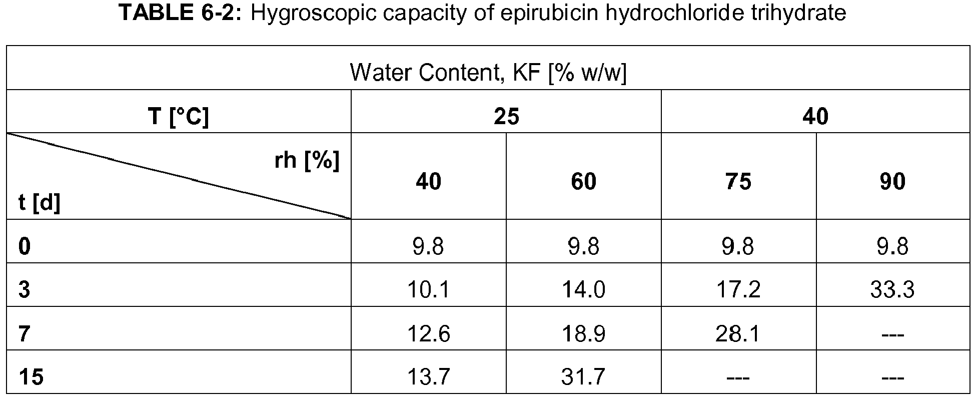

The data show a considerable extent of water absorption already after 3 days of exposure. Hence, again, the superior properties of the crystalline epirubicin hydrochloride of the present invention are an inherent feature of the monohydrate form obtained.

The present invention illustratively described herein may suitably be practiced in the absence of any element or elements, limitation or limitations, not specifically disclosed herein. Thus, for example, the terms "comprising", "including", "containing", etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the invention claimed. Thus, it should be understood that although the present invention has been specifically disclosed by embodiments and optional features, modifications and variations of the inventions embodied therein may be resorted to by those skilled in the art, and that such modifications and variations are considered to be within the scope of this invention.

The invention has been described broadly and generically herein. Each of the narrower species and sub-generic groupings falling within the generic disclosure also form part of the invention. This includes the generic description of the invention with a proviso or negative limitation removing any subject matter from the genus, regardless of whether or not the excised material is specifically recited herein.

Other embodiments are within the following claims. In addition, where features or aspects of the invention are described in terms of Markush groups, those skilled in the art will recognize that the invention is also thereby described in terms of any individual member or subgroup of members of the Markush group.

## Claims

1. Method for producing crystalline epirubicin hydrochloride, comprising:
(a) adding at least a first solvent and at least a second solvent to epirubicin hydrochloride, wherein the first solvent is a linear or branched C₄ to C₅ alcohol, and the second solvent is a linear or branched C₂ to C₃ alcohol;
(b) adjusting in the solution obtained in step (a) the water content to an amount in the range between 7% and 12% (w/w); and
(c) heating the solution obtained in step (b) to a temperature of 70°C to 90°C in order to allow crystallization;
wherein the crystalline epirubicin hydrochloride produced is a monohydrate having a water content in the range between 2.7% and 3.5% (w/w) and being devoid of residual solvents.

2. The method of claim 1, wherein the water content in step (b) is adjusted to an amount in the range between 8% and 11% (w/w).

3. The method of claim 1 or 2, wherein the first solvent is selected from the group consisting of 1-butanol, 2-butanol, isobutanol, tert-butanol, and mixtures thereof; and wherein the second solvent is selected from the group consisting of 1-propanol, isopropanol, ethanol, and mixtures thereof.

4. The method of claim 3, wherein the first solvent is 1-butanol and the second solvent is 1-propanol.

5. The method of any one of claims 1 to 4, wherein the volume ratio of the first solvent to the second solvent is in the range between 1:1 and 2:1 (v/v).

6. The method of any one of claims 1 to 5, wherein the final concentration of epirubicin hydrochloride in the solution of step (b) is in the range between 10 g/l and 100 g/l.

7. The method of any one of claims 1 to 6, wherein the solution in step (c) is heated to a temperature of 75°C to 85°C.

8. The method of any one of claims 1 to 7 further comprising: seeding of the solution obtained in step (b) in order to induce crystallization.

9. The method of any one of claims 1 to 8, further comprising: purifying the crystals obtained in step (c).

10. The method of any one of claims 1 to 9, wherein the water content of the crystalline monohydrate of epirubicin hydrochloride is in the range between 2.8% and 3.3% (w/w).

11. Crystalline monohydrate of epirubicin hydrochloride, being produced by the method of any one of claims 1 to 10.

12. Crystalline monohydrate of epirubicin hydrochloride, having a water content in the range between 2.7% and 3.5% (w/w) and being devoid of residual solvents.

13. The crystalline monohydrate of epirubicin hydrochloride of claim 12, having a water content in the range between 2.8% and 3.3% (w/w).

14. The crystalline monohydrate of epirubicin hydrochloride of claim 12 or 13, further **characterized by** a powder X-ray diffraction pattern comprising peaks at average diffraction angles (2Θ) of 5.1°, 9.1°, 13.6°, 22.1°, 22.5°, and 24.0° (each ± 0.2°).

15. The crystalline monohydrate of epirubicin hydrochloride of any one of claims 11 to 14 for use in the prevention and treatment of cancer.
